# EUROPEAN PATENT APPLICATION

(11) **EP 1 342 765 A2**
(43) Date of publication of application: **10.09.2003**
(21) Application number: 03004433.3
(22) Date of filing: 27.02.2003
(51) Int. Cl.: C09J 153/02

(54) **Hot melt pressure sensitive adhesives for disposable articles**

(30) Priority: 08.03.2002 US 93616
(71) Applicant: Henkel Kommanditgesellschaft auf Aktien, 40589 Düsseldorf (DE)
(72) Inventor: Hatfield, Steve, Corinth, TX 76210 (US)

(57) **Abstract**

A hot melt pressure sensitive adhesive (HMPSA) is obtained by combining a linear A-B-A block copolymer, wherein the B component is polyisoprene and the A component is polystyrene (S-I-S), at least one compatible tackifying resin, at least one plasticizer, antioxidant(s) and stabilizer(s) and optionally a wax, wherein the A-B-A block copolymer contains 0 to 10 % by weight residual A-B diblock. The HMPSA is suitable as a positioning adhesive for disposable articles like plasters, bandages, sanitary napkins, e. g. feminine napkins, adult incontinent pads or diapers. The HMPSA has a viscosity from 3,500 mPas to 25,000 mPas at 140°C and exhibits adhesion values from 1.0 to 3.0 N / cm at coat weights of 10 to 25 grams per square meter.

## Description

### Background of the Invention

### Field of the Invention

The present invention relates to hot melt pressure sensitive adhesives (HMPSA) based on linear A-B-A block copolymers, wherein the B component is polyisoprene and the A component is a polyvinylaromatic such as polystyrene (S-I-S), and their use for disposable articles which are adhesively adhered to a substrate.

### Discussion of the Related Art

A hot melt adhesive is in general a nearly water- and solvent-free adhesive which is applied on the substrate out of a molten state. The setting of the adhesive happens during the cooling down phase of the melt. A hot melt pressure-sensitive adhesive (HMPSA) is an adhesive that retains high surface tackiness over time. In other words, a HPMSA has a theoretically infinite open time. The bonding force of the HPMSA is derived from the ability of the adhesive to be compatible with the surface of both the substrate and the bonded materials. The adhesive bond arises from the compatibility of the adhesive mass with both the object and the substrate and the internal cohesiveness of the adhesive mass.

Removable or releasable HMPSA's for disposable articles like hospice care such as plasters or bandages, hospital absorbent bed pads, feminine hygiene articles such as sanitary napkins, panty liners or absorbent pads (referred to as Femcare Products), adult incontinent pads, diapers and the like are described as "garment attachment", "fixation" or "positioning" adhesives. Positioning adhesive are e. g. used to secure a hygiene article to the undergarment or bedding to keep it in place until removal. In particular a positioning HMPSA is permanently adhered to at least one substrate of the disposable article and removably or releasably attachable to another substrate.

Over the past 20 years, there has been a need for the companies manufacturing Femcare Products to use lower amounts of the forementioned positioning adhesives to lower costs and to make the resulting constructions more flexible and comfortable for the end user.

Several US patents (e. g. 4,166,464; 4,608,047; 4,900,320; 4,911,701; 5,281,209; 6,319,239) outline this increasing complexity in the requirements of Femcare Products. Several patents also illustrate the development of hot melt positioning adhesives for disposable articles.

Basically, in 1979 with the granting of Collins et al., "Absorbent Article with adhesive strip", US 4,136,699, the use of a formulated SEBS tri-block polymer and/or SBS radial polymer based hotmelt for use as an adhesive strip with a removable release liner for sanitary napkins was first described. These hotmelts are characterized by having a viscosity between 500 and 15,000 mPas at 150°C, good stability, high cohesive strength and moderate to low tack and therefore required high add-on weights to remain secure. This patent further taught that conventional SIS polymers of the time, such as Kraton® 1107, did not possess the necessary chemical stability properties, nor the specific adhesion requirements to be formulated into usable positioning formulations of this viscosity range. Before this invention, mostly solvent and water-based acrylate type adhesives were utilized for these applications; both of these types had severe processing limitations such as drying and wicking problems on the backsheet of said absorbent pads.

Solvent-type pressure sensitive adhesives are in use also nowadays. For instance, solvent-type re-peelable pressure sensitive adhesives based on block copolymers comprising a polymer block A consisting mainly of units derived from a vinylaromatic compound and a polymer block B consisting mainly of units derived from a conjugated diene compound, wherein said copolymers have a block A content of 18 % by weight or higher and/or have a degree of coupling of 90 % or higher are described in EP 0683216.

Chen et al., US 4,460,364, made further improvements on the SEBS based hotmelts, by defining the MW of the polymer and rheological properties of the resulting HM. However, positioning adhesives based on SEBS formulations typically are applied in amounts above 25 grams per square meter.

In US 5,863,977, Fischer et al. describe the use of high molecular weight (i.e. melt indexes below 1, best known as fractional melt index polymers) SEBS A-B-A tri-block copolymers for use in positioning adhesives. However, in practice the viscosity of the resulting hot melt is difficult to control and therefore the resulting properties such as cohesive strength and tack vary greatly from production lot to lot and from polymer lot to lot.

Furthermore, several patents describe the use of SIS copolymers as the basis for construction hot melts for disposable consumer articles such as diapers, sanitary napkins, panty liners, bed pads and the like.

The patents of Diehl et al., US 5,266,394 and US 5,750,623, describe the use of linear low di-block SIS tri-block copolymers for use as construction hot melts for disposable articles.

US patent 5,523,343 by Giordano et al. describes a pressure sensitive hot melt adhesive composition based on styrene-butadiene radial block copolymers, styrene-isoprene linear and/or radial block copolymers, and plasticizing oils which maintain low tack values (i.e. peel adhesion and quick stick) while having a good balance of properties normally required. While these compositions are interesting from an experimental viewpoint, they are very high in viscosity and not viable adhesives for typical disposable article positioning applications which require low viscosities (i.e. < 10,000 mPa•s) at 140°C.

Although a broad state of the art for HMPSA's already exists, there is thus a need for hot melt pressure sensitive adhesives with improved properties. The use of lower amounts (lower add-on levels) of positioning adhesives, preferably down to about 10 to 15 grams adhesive/m², in the manufacturing of disposable articles requires an increased need for higher tack, preferably adhesion values in the range of 1.5 to 2.5 N/cm, to still maintain the hold required for the article to properly function. In addition, higher cohesive strength is required to prevent transfer of the adhesive to undergarments, especially when the adhesive is aged. The requirement of improved transfer resistance is connected additionally with improved high temperature resistance. Low viscosity, high tack hot melt positioning adhesives with improved adhesion to polyolefins are needed, e. g., for the direct application at low temperatures to the backsheet of sanitary napkins and the like. The backsheets of sanitary napkins are usually made of polypropylene or polyethylene; these are materials which distort at temperatures above 130 °C.

Application using modern high speed machines requires adhesives with improved mileage versus current technologies, allowing manufacturers to safely apply from about 10 to about 15 grams per square meter of adhesive and still maintain the necessary minimum tack levels of about 1.5 N/cm.

### Summary of the Invention

The solution to the problem stated above is defined in the claims and involves providing a hot melt pressure sensitive adhesive (HMPSA) comprising:
(a) about 10 percent to about 40 percent, preferably about 15 percent to about 35 percent, based on the weight of the HMPSA, of a linear A-B-A block copolymer,
   wherein the B component is polyisoprene and the A component comprises one or more vinylaromatic compounds in polymerized form, preferably polystyrene (S-l-S);
(b) about 40 percent to about 70 percent by weight of at least one compatible tackifying resin, based on the weight of the HMPSA;
(c) about 10 percent to about 40 percent by weight of at least one plasticizer, based on the weight of the HMPSA;
(d) about 0 percent to about 10 percent by weight of a wax, based upon the weight of the HMPSA; and
(e) about 0.1 percent to about 2.5 percent by weight of antioxidant(s) and stabilizer(s), based upon the weight of the HMPSA; and
wherein the A-B-A block copolymer contains 0 to 10 %, preferably 0 to 5 %, and most preferably 0 to 1 % by weight residual A-B diblock.

The block copolymers used in the present invention are linear block copolymers, having the general configuration: A-B-A block copolymer, wherein the B component is polyisoprene and the A component is a vinylaromatic compound (or mixture of vinylaromatic compounds) in polymerized form, preferably polystyrene (S-l-S). The polymer blocks A are non-elastomeric polymer blocks which, as homopolymers, have glass transition temperatures above 20 degrees Centigrade. The elastomeric blocks B are comprised of isoprene-containing polymer.

The polyvinylaromatic portion (component A) of said block copolymers comprises 15 percent to about 50 percent, preferably 15 percent to 35 percent, by weight of the A-B-A block copolymer. The A-B-A block copolymer is further characterized by having a melt flow index of 3 to 50 g/10 min, preferably 5 to 20 g/10min, determined by ASTM-d 1238-95.

Block copolymers useful for the invention herein are styrene-isoprene-styrene tri-block copolymers. Each styrene block preferably has a molecular weight ranging from about 1000 to 75000, more preferably from 5000 to about 50000, most preferably from 8000 to 20000. The isoprene block has typically a molecular weight of about 25000 to 300000, more preferably between 30000 and 250000 and most preferably between about 350000 and 200000. Suitable SIS copolymers of this type are obtainable from Dexco under the tradenames, VECTOR 4111 D, 4211 D and 4411 D. They can be used in blends with other SIS copolymers like KRATON D1107, D1111, D1112, D1113X, D1114X or SolT190, SolT193A, SolT193B and SolTE-9308 from Enichem. The A-B-A block copolymer, containing 0 to 10 % by weight residual A-B diblock, is present in these blends in an amount from 20 to 99 %, preferably 40 to 99%. Blends of different A-B-A block copolymers may be used to advantage. In one preferred embodiment of the invention, a mixture of an S-l-S block copolymer having a relatively low polystyrene content (preferably, 15 weight % to 22 weight %) and an S-l-S block copolymer having a relatively high polystyrene content (preferably, 26 weight % to 32 weight %) is utilized. The weight ratio of low polystyrene S-l-S block copolymer to high polystyrene S-l-S block copolymer is preferably in the range of from 1:2 to 2:1, more preferably, 1:1.2 to 1.5:1.

The compatible tackifying resins useful in the adhesive compositions, from about 40 % to about 70 %, preferably 45 % to 65 %, by weight, are preferably selected from the group: aliphatic petroleum resins and the hydrogenated derivatives thereof, aromatic petroleum resins and the hydrogenated derivatives thereof, aliphatic/aromatic petroleum resins and the hydrogenated derivatives thereof, hydrocarbon resins, styrene resins, alpha- methyl styrene resins, polyterpene resins, copolymers and terpolymers of natural terpene resins, pentaerythritol esters of wood, gum, and tall-oil rosins, glycerol esters of wood, gum, and tall-oil rosins, and mixed esters of rosins and mixtures. More particularly , the useful tackifying resins include any compatible resins or mixtures of said resins from the following group:
(1) natural and modified rosins such as, for example, gum rosin, wood rosin, tall oil rosin, distilled rosin, hydrogenated rosin, and polymerized rosin;
(2) glycerol, pentaerythritol (PE), and mixed glycol esters of natural and modified rosins such as, for example, the glycerol esters of wood rosin or tall oil rosin, the PE ester of hydrogenated rosin, and the phenolic-modified PE ester of rosin having a softening point, as determined by ASTM method E28-58T, of about 30°C to about 125°C;
(3) copolymers and terpolymers of terpenes, e.g. styrene-terpene and alpha-methyl styrene-terpene, having a softening point from 60°C to about 125°C;
(4) polyterpene resins having a softening point of from about 10°C to about 125°C;
(5) phenolic-modified terpene resins and the hydrogenated derivatives thereof having a softening point of about 60°C to about 130°C;
(6) aliphatic petroleum hydrocarbon resins having a softening point of about 5°C to about 140°C;
(7) alicyclic petroleum hydrocarbon resins and the hydrogenated derivatives thereof; and
(8) endblock reinforcing resins based on homopolymers, copolymers, and terpolymers of styrene, alpha-methylstyrene, vinyl toluene, and phenylene oxide having a softening point from 70°C to 165°C. "Endblock resins" are substantially aromatic and compatible with the endblock "A" of the A-B-A copolymer. The preferred tackifying resins for the present invention are
(9) hydrogenated or partially hydrogenated cycloaliphatic petroleum hydrocarbon (DCPD) resins and C5 hydrocarbon resins having softening points from about 60°C to about 130°C. Useful resins include Eastotac® H-115 W and more preferably Eastotac® H-100 W. These resins are available in the E grade, R grade, L grade and W grade indicating increasing levels of hydrogenation and therefore lighter colour respectively. They are available from Eastman Chemical Co. in Kingsport, Tenn. Other available resins include Escorez® 5300 and Escorez® 5400, partially hydrogenated cycloaliphatic petroleum hydrocarbon resins, and Escorez® 5600, a partially hydrogenated aromatic modified petroleum hydrocarbon resin, all having softening points of about 100 °C and available from Exxon Chemical Company in Houston, Texas; Wingtack® Extra which is an aliphatic, aromatic petroleum hydrocarbon resin, Wingtack® 86, an aromatic modified synthetic polyterpene hydrocarbon resin and Wingtack® 95, a synthetic polyterpene, all having softening points of less than about 100° C and available from Goodyear Tire and Rubber Co. in Akron, Ohio and Arkon® P-70, P-90 and P-100, synthetic petroleum hydrocarbon resins having softening points of 70 °C, 90 °C and 100 °C respectively and available from Arakawa Chemical (USA) Inc. in Chicago, Illinois. Further examples include Hercolite® 2100 which is a partially hydrogenated cycloaliphatic petroleum hydrocarbon resin available from Hercules, Inc. in Wilmington, Delaware, and Zonatac® 105 Lite which is a styrenated terpene hydrocarbon resin, made from d-limonene and available from Arizona Chemical in Panama City, Florida.
These tackifying resins offer excellent compatibility with styrene-isoprene-styrene copolymers and their light color and low odor make them ideal for consumer article construction applications.

Various plasticizers are also present in the hot melt adhesives based on the teachings of the present invention, in amounts from about 10 % to about 40 %, preferably about 15 % to about 35 % by weight, primarily to provide for viscosity control and wetting and specific adhesion to the coated substrates. These plasticizers are preferably selected from the group consisting of naphthenic oil, phthalate and adipate esters, oligomers of polypropylene, polybutenes, polyisoprene, hydrogenated polyisoprene and polybutadiene, benzoate esters, and vegetable and animal oils and derivatives thereof. Fully hydrogenated plasticizers can be selected from paraffinic hydrocarbon oils - available, for example, under the tradename Primol from Exxon -, polyisobutylenes, poly-1-butene oils or hydrogenated naphthenic oils. Preferably, the polyisobutylenes have a molecular weight ranging from about 600 to 5000, more preferably from 800 to 4000. Typically they are highly viscous liquids at room temperature. Suitable polyisobutylenes are available under the tradename "Parapol" from EXXON Chemicals or under the tradename "Oppanol" from BASF. Prefered plasticizers are paraffinic oils with a viscosity of 100 to 600 mPas, preferably 150 to 300 mPas at 25 °C. They are available from Witco under the tradename of Kaydol oil or its technical equivalent, Primol 352 from the Esso Company.

Waxes in the composition of the present invention ranging from 0% to 10% by weight may be used to reduce the surface tack of the adhesives without appreciably decreasing their adhesive bonding characteristics. These waxes also are used to reduce the blocking of the composition without affecting the temperature performance. These waxes are especially useful for multi-purpose hot melt pressure sensitive adhesives, in that these components offer additional wetting and added creep resistance for foamed elastic attachments and the like. These waxes can be selected from the group comprised of paraffin wax, microcrystalline wax, Fischer-Tropsch wax, polyethylene wax, ethylene vinyl acetate wax, oxidized polyethylene wax, hydrogenated castor oil wax and derivatives thereof, and polypropylene wax. Among the useful waxes are low molecular weight, that is, 1000-6000, polyethylene having a hardness value, as determined by ASTM method D- 1321, of from about 0.1 to 120 and ASTM softening points of from about 65°C to 120°C. Also, petroleum waxes may be used such as paraffin wax having a melting point of from about 55°C to 75°C and microcrystalline wax having a melting point of from about 55°C to 95°C, the latter melting points being determined by ASTM method D 127-60. Atactic polypropylenes are also suitable; typically, they have a ring and ball softening point of from about 120 °C to 160 °C. Other suitable waxes include synthetic waxes made by polymerizing carbon monoxide and hydrogen such as Fischer-Tropsch wax and polyolefin waxes. As used herein, the term "polyolefin wax" refers to those polymeric or long-chain entities comprised of olefinic monomer units. These materials are commercially available from Eastman Chemical Co. under the trade name "Epolene." The materials which are preferred for use in the compositions of the present invention have a ring and ball softening point of 95°C to 175°C. Each of these wax diluents is solid at room temperature. Other useful substances include hydrogenated animal, fish and vegetable fats and oils such as hydrogenated tallow, lard, soya oil, cottonseed oil, castor oil, menhadin oil, cod liver oil, etc., and which are solid at ambient temperature by virtue of their being hydrogenated, have also been found to be useful with respect to functioning as a wax diluent equivalent. These hydrogenated materials are often referred to in the adhesives industry as "animal or vegetable waxes." The preferred waxes for the present invention are selected from oxidized polyethylene waxes.

Stabilizers or antioxidants utilized for the present invention include high molecular weight hindered phenols and multifunctional phenols. Hindered phenols are well known to those skilled in the art to be effective primary stabilizers for styrene-isoprene block copolymers. Typical commercially available stabilizers of these types are supplied by Ciba-Geigy under the tradenames Irganox 1010 and Irganox 1076. Useful secondary stabilizers include phosphites, such as tris-(p-nonylphenyl)-phosphite (TNPP) and bis(2,4-di-tert-butylphenyl)4,4'-diphenylene-diphosphonite and di-stearyl-3,3'-thiodipropionate (DSTDP). Stabilizer(s) can represent from 0.1 to about 2.5, preferably from about 0.25 to about 1.5, % by weight of the hot melt pressure sensitive adhesive of this invention.

Other optional ingredients may also be added to the adhesives of the present invention providing that these ingredients do not adversely affect the removability and nonstaining characteristics of the adhesive. Such other ingredients may include other block copolymers; homopolymers, copolymers and terpolymers of ethylene including ethylene vinyl acetate copolymers, ethylene n-butyl acrylate copolymers and ethylene methacrylate copolymers; interpolymers of ethylene having at least one C₃ to C₂₀ alpha-olefin and homopolymers, copolymers and terpolymers of propylene to mention only some examples.

In addition, proper manufacturing procedures of the adhesive are required to assure product consistency. These procedures include mixing of the components of the adhesive under vacuum to remove air, which can cause oxidation and a loss of physical properties. Breaking the vacuum with nitrogen or carbon dioxide gas to prevent oxygen entrapment in the adhesive is also desirable. Using a minimum amount of thermal and mechanical energy to manufacture the adhesive and cooling the finished adhesive quickly to prevent degradation are additional useful procedures to employ. The hot melt pressure sensitve adhesive of this invention may be prepared by mixing the components in a heated tank under vacuum at a temperature of from about 120° C. to about 190° C. until a homogeneous blend is obtained. Minimal amounts of thermal and mechanical energy should be employed to manufacture the adhesive. The determination of such amounts are well within the experience of one skilled in the art. This step usually requires two to three hours. The vacuum is then broken with nitrogen or carbon dioxide gas to prevent air entrapment in the adhesive. The finished adhesive is then packed and cooled for shipment to the manufacturer of the disposable articles.

Disposable articles according to the invention, wherein at least one substrate is continuously or discontinuously coated with the hot melt pressure sensitive adhesive of the present invention, comprise:
i) a polyolefin or nonwoven substrate (i), and
ii) a pressure sensitive adhesive (ii) applied onto substrate (i), said adhesive comprising:
   (a) about 10 percent to about 40 percent based on the weight of the HMPSA, of a linear A-B-A block copolymer, wherein the B component is polyisoprene and the A component is a polymerized vinyl aromatic compound such as polystyrene (S-l-S);
   (b) about 40 percent to about 65 percent by weight of at least one compatible tackifying resin, based on the weight of the HMPSA;
   (c) about 10 percent to about 40 percent by weight of at least one plasticizer, based on the weight of the HMPSA;
   (d) about 0 percent to about 10 percent by weight of a wax, based upon the weight of the HMPSA; and
   (e) about 0.25 percent to about 2.0 percent by weight of antioxidant(s) and stabilizer(s), based upon the weight of the HMPSA;
   wherein the A-B-A block copolymer contains 0 to 10 % by weight residual A-B diblock;
iii) wherein said adhesive (ii) bonds substrate (i) to at least one elastic, polyolefin, foam, or nonwoven substrate.

In a preferred embodiment, the disposable article is coated with a pressure sensitive adhesive (ii) comprising:
(a) about 10 percent to about 40 percent, based on the weight of the HMPSA, of a linear A-B-A block copolymer, wherein the B component is polyisoprene, the A component is polystyrene (S-I-S); wherein, the A component is present in an amount of about 18 percent to about 30 percent by weight of the A-B-A block copolymer, the A-B-A block copolymer has a melt flow index of 6 to 20 and the A-B-A block copolymer contains 0 to 5 % by weight residual A-B diblock;
(b1) about 40 percent to 70 percent of a hydrogenated aliphatic/aromatic tackifying resin;
(b2) about 0 percent to 10 percent of an endblock reinforcing resin, such as a alpha- methylstyrene resin;
(c) about 10 percent to 40 percent of paraffinic oil plasticizer;
(d) about 0 to 5 parts of an oxidized polyethylene wax or polyethylene wax;
(e) about 0.25 percent to about 2.0 percent by weight of antioxidant(s) and stabilizer(s);

In a further preferred embodiment of the invention, the disposable article is coated with a pressure sensitive adhesive (ii) having a viscosity of 3,600 mPas to 10,000 mPa.s at 140°C and adhesion values from 1.0 to 3.0 N/cm at coat weights of 10 to 20 grams per square meter.

Disposable articles are preferably selected from the group consisting of plasters, bandages, sanitary napkins, e. g., feminine napkins, adult incontinent pads and diapers. The most preferred embodiment of the invention is a sanitary napkin or the like article for adhering to the crotch portion of an undergarment, comprising: an adsorbent body for absorbing body fluids and having a body facing side and a garment facing side and wherein a layer of the pressure sensitive adhesive (ii) at a coat weight of 10 to 20 grams per square meter overlying at least a portion of the garment facing side, whereby said sanitary napkin may be applied to said undergarment and wherein the pressure sensitive adhesive will exhibit an adhesion value of 1.5 to 3.0 N/cm and a transfer of adhesive of less than 1 milligram per square meter after being applied to cotton and conditioned for 24 hours at 40°C with 1 kg of pressure.

The manufacturer of the disposable articles then re-melts the hot melt adhesive for use as the positioning adhesive in said articles. The application of the hot melt pressure sensitive adhesive may be accomplished by several applications systems depending on the type of article being made and the substrates involved. The pressure sensitive hot melt is preferably applied by a method selected from the group consisting of transfer coating from silicone release paper and/or film, slot-die extrusion, fiber melt deposition, melt blown techniques (i.e. spiral spray and the like), and pattern coating methods. These application systems may involve: Spiral spray techniques, multi-line extrusion, multi-dot pattern printing, slot die extrusion, and melt blown fiber deposition. The hot melt pressure sensitive adhesives produced by the teachings of the present invention can be applied by any of the forementioned techniques for the manufacture of disposable articles, and wherein the said adhesive preferably is bonding a polyolefin or nonwoven substrate to at least one elastic, polyolefin, foam, or nonwoven substrate, most preferably wherein the hot melt pressure senstive adhesive is applied to the backsheet of a Femcare Product for use as a fixation or positioning adhesive. In a preferred embodiment of the invention, the pressure sensitve hot melt is applied directly to a polyolefin substrate via slot-die extrusion, fiber melt deposition, and/or spiral spray techniques.

The properties of the HMPSA of the present invention clearly surpass those of current state of the art HMPSA's with the following key features:
- No solvent, either water nor any organic solvent, is necessary for the manufacturing and application of the HMPSA;
- Low viscosity ranges from 3,500 mPas to 25,000 mPas and especially from 3,800 mPas to 15,000 mPas, at 140°C;
- High tack, especially high initial peel to cotton. Adhesion values range from 1.0 to 3.0 N / cm and especially from 1.5 to 2.5 N/cm at coat weights of 10 to 25 grams and especially of 12 to 18 grams per square meter;
- Improved adhesion to polyolefins and backsheets at low temperatures, particularly at a sub-ambient temperature of 4 °C;
- No transfer of adhesive to the substrate even after long term exposure to UV light;
- No transfer of adhesive to the substrate even when aged for 24 hours at 40°C with 1.0 kg pressure to cotton cloth;
- Direct application, e. g., to the backsheet of a Femcare Product, is possible, instead of transfer coating from silicone release liner;
- High thermal stability. Even after exposure at 150 °C for 48 hours followed by 16 hours at 160 °C only minimum changes in viscosity and softening point are observed.

This invention can be further illustrated by the following examples of the preferred embodiments thereof, although it will be understood that these examples are included only for illustration and comparison to the existing art, and are not intended to limit the scope of the invention unless specifically indicated.

### I. Examples

### Example 1:

Example 1 is an illustration of the present invention based on linear low di-block SIS triblock polymer Vector® 4111D, supplied by Dexco, having 17.5 to 19 weight % styrene and a MFI of 9.7 to 13.8 with a di-block content below 1%; a hydrogenated DCPD resin from Exxon (Escorez® 5400) with a Tg of 54°C and a RBSP of 103°C; a hydrotreated paraffinic white oil, from Esso (Primol® 352) and a hindered phenolic type anti-oxidant from Ciba-Geigy (Irganox® 1010). Properties include excellent tack, resistance to transfer (no residue) and good aging properties.

### Example 2:

Example 2 is based on the present invention and demonstrates the formulation window of Example 1.

### Example 3:

Example 3 is based on Examples 1 and 2, but illustrates the affect of adding an endblock reinforcing resin such as Kristalex® F-100, modified alpha-methyl styrene resin with a RBSP of 100°C, supplied by Eastman Chemical. Properties include excellent tack, resistance to transfer (no residue) and good aging properties.

### Example 4:

Example 4 is also based on the present invention, however using a higher styrene content (from 28 to 31 weight %) linear low diblock (< 1% diblock) SIS with a MFI of 10 to 16, from Dexco, supplied under the name Vector® 4211. Properties include excellent tack, resistance to transfer (no residue) and good aging properties.

### Example 5:

Example 5 is also based on the present invention. It is based on a blend of the nominally 18 weight % styrene content SIS, (Vector® 4111) and a 30% styrene content SIS, Vector 4211, both of which are linear low di-block (<1% Sl) SIS copolymers. The resulting formulation gives a hot melt with excellent tack, an excellent balanced peel from cotton and leaves no residue behind after aging at 40°C.

### Example 6 (comparative):

Example 6 is a prior art SBS formulation based on a linear high styrene (41 to 45 weight %) SBS (Vector® 4461-D), a blend of tackifying resins (one being Escorez® 5320 from Exxon Chemical, a hydrogenated C5 resin with a RBSP of 120° and the other being a styrenated terpene resin from Arizona Chemical, called Sylvares® ZT-105-LT, with a RBSP of 105°C), an endblock reinforcing resin (Kristalex® F-100) white oil (Primol® 352) and anti-oxidant (Irganox® 1010). A large amount of build up in adhesion after aging is noticeable.

### Example 7 (Comparative):

Example 7 is a prior art SEBS formulation as described in US patents 4,136,699 and 4,460,364. The formula is based on a SEBS triblock polymer (Kraton® G-1652, nominally 28% styrene), white oil, Escorez® 5400 and anti-oxidant (Irganox® 1010). Low peel values to cotton at 20 g/m² coat weights were observed. These products are very stable but still lack the necessary tack and adhesion to meet the higher end use requirements of the lower coat weights.

**Table 1:**

| MATERIAL | Expl. 1 | Expl. 2 | Expl. 3 | Expl. 4 | Expl. 5 | Expl. 6 | Expl. 7 |
|---|---|---|---|---|---|---|---|
| Vector® 4111 | 22 | 21 | 20 | 0 | 12 | 0 | 0 |
| Vector® 4211 1 | 0 | 0 | 0 | 23 | 10 | 0 | 0 |
| Kraton® 1652 | 0 | 0 | 0 | 0 | 0 | 0 | 16 |
| Vector® 4461 | 0 | 0 | 0 | 0 | 0 | 20 | 0 |
| Primol® 352 | 25 | 23 | 24 | 22 | 23 | 23 | 29 |
| Escorez® 5400 | 52.2 | 55.2 | 52.2 | 54.2 | 54.2 | 0 | 54.2 |
| Escorez®5320 | 0 | 0 | 0 | 0 | 0 | 22.5 | 0 |
| Sylvares® ZT-105-LT | 0 | 0 | 0 | 0 | 0 | 22.5 | 0 |
| Kristalex ® F-100 | 0 | 0 | 3 | 0 | 0 | 10 | 0 |
| Irganox® 1010 | 0.8 | 0.8 | 0.8 | 0.8 | 0.8 | 1.0 | 0.8 |
| All values are in percent by weight. Expl. = Example | | | | | | | |

### II. Test Methods

### II-1 Viscosity

The melt viscosities of the adhesives are determined according to ASTM-D 3236-88. Equipment: Viscometer - Brookfield RVT with spindles (stainless steel), type SC 4 - 21/SC 4 - 27/SC 4 - 28/SC 4 - 29

### II-2 Ring and Ball Softening Point (RBSP)

The Ring and Ball softening points of the tackifying resins and adhesives are determined according to ASTM-E 28-67.

### II-3 Residue Test

Determination of residues after simulating the use of a sanitary napkin (pressure, temperature and time) on cotton cloth and after removal from the cotton cloth.

### Equipment:

1) Cotton Cloth: Schiesser No. 4467 (washed on 95 °C and air dried)

1) Ventilated oven capable of maintaining 40°+/- 1°C
2) An acrylic glass plate (6 cm by 3 cm)
3) A one kg weight

### Sample preparation / measurement:

1) The positioning strip is cut from a sanitary napkin and the siliconized paper is removed.
2) A piece of cotton is placed on the positioning adhesive.
3) On the cotton cloth with positioning strip an acrylic glass plate and a weight of 1 kg is placed.
4) The finished sample (3 samples needed) is placed into the ventilated oven at 40 °C for 4 hrs and then 24 hours.
5) Then the sample is removed from the oven, the weight is removed also and the sample needs 1 hr to cool down.
6) After cooling down the cotton will be removed manually and the cotton is examined for residues.

### II-4 Aging Test / Peel Test:

The accelerated aging test is a method to simulate a long term storage by increasing the storage temperature and storing only for some weeks. A storage at 60 °C for 2 weeks approximately simulates a shelf storage of 48 weeks at 20 °C.

### Equipment:

1) Cotton Cloth: Schiesser No. 4467 (washed on 95 °C and airdried)
2) Tensile tester Zwick Type 1435
3) An Atlas - Brabender press roller (5 lbs / 1 inch per sec)
4) A climatized room (23 °C and 50 % humidity)

### Sample preparation / measurement:

1) The whole positioning strip is cut from a napkin and stored in a climatized room for 24 hrs.
2) The siliconized paper is removed and placed on the positioning adhesive without pressing.
3) Thereafter, the prepared sample is passed through the Brabender roller.
4) The sample is fixed in the jaws of the tensile tester and it will be pulled with a speed of 300 mm/min. Strength is calculated in N/cm and in N/pad.
5) The above procedure is repeated after aging 2 weeks at 60°C.

### II-5 Coat Weight Determination

The purpose of the coat weight determination is to find the amount of adhesive applied to the PE or PP film used to make the sanitary napkin.

### Sample preparation / measurement:

1) The positioning strip is cut from a sanitary napkin and the siliconized release paper is removed.
2) The initial weight of the sample is measured.
3) Then the adhesive is removed with hexane by using a paper towel. After drying the sample in an oven (∼ 70 °C), the sample is reweighed. The difference in weights is the amount of adhesive and is calculated in g/m² based on the area of the positioning strips.

### II-6 180° Peel Test

This method is designed to test the adhesion of pressure sensitive adhesives to stainless steel panels or HDPE panels, but has been modified here for adhesive coated on PE applied to cotton cloth.

This method is similar to PSTC-1 and ASTM-D 3330

### Equipment:

1) Tensile tester Zwick Type 1435
2) 50 micron Polyethylene film.
3) Release paper.
4) Acumeter 5 cm laboratory coater.
5) Roll down device with 2 kg roller.
6) 5 cm X 15 cm Cotton Cloth strips.
7) 5 cm X 15 cm HDPE panels.

### Sample:

1) Samples are coated onto silicone release paper and transferred to 50 micron PE film at 15 micron coat weight +/- 3 micron and nipped with release paper with a hand roller.
2) Test strips are 2.5 cm wide and approximately 30 cm long.
3) Test samples are conditioned for at least 24 hours in standard conditions.

### Procedure:

1) Clean panel three times. First wipe gross adhesive with a suitable solvent and a lint free towel (Suitable solvents are toluene, n-heptane, MEK). Repeat cleaning with new towel and methanol. Then clean any residue with a clean towel and hexane.
2) Set up Instron to track tension in the anticipated range and adjust zero. Set crosshead speed to 30 cm/min. Set up chart recorder to appropriate range and adjust zero.
3) Remove release paper backing from one end of test strip and touch to one end of panel. Roll strip down to panel using only the weight of the roller and moving at a rate of 30 cm/min. At the same rate, roll the roller backward to the start and off.
4) Test sample immediately - within one minute. Fold back the free end of the strip and peel approximately 2.5 cm from the panel. Clamp that end of the panel to the lower jaw of the Instron. Clamp the free end of the test strip the to upper jaw. Start the upper jaw in motion. Disregard the values obtained from the first 2.5 cm of peel. Use the average force value obtained during the next 5 to 8 cm as the adhesion force.

### Report:

1) Report material of substrate panel.
2) Report the peel adhesion force in N/cm (width).

### II-7 Adhesion to HDPE at 4°C

This method is designed to evaluate an adhesive's relative adhesion to high density polyethylene at low temperatures.

### Equipment:

1) 50 micron PE film.
2) Siliconized Release Paper.
3) 5 cm X 12 cm HDPE panels.
4) Acumeter 5 cm laboratory coater.
5) Roll down device with 2 kilogram rollers.
6) Refrigerated cabinet maintained at 4°C.

### Sample:

1) Samples are coated onto silicone release paper and transferred to 50 micron PE film at 15 micron coat weight +/- 3 micron and nipped with release paper with a hand roller.
2) Test samples are conditioned for at least 24 hours at standard conditions. Procedure:

1) Cut 2.5 cm X 20 cm strips from coated stock.
2) Clean panels three times. First wipe gross adhesive with a suitable solvent and a lint free towel (suitable solvents are toluene, n-heptane, MEK). Repeat cleaning with new towel and methanol. Then clean any residue with a fresh towel and hexane.
3) Remove release paper backing from one end of the test strip and touch to one end of the test panel. Roll strip down to panel using only the weight of the roller and moving at a rate of 30 cm/min. At the same rate, roll the roller backward to the starting position and remove the panel.
4) Condition the test samples for one hour after roll down at standard conditions.
5) Place test samples in refrigerated cabinet at 4°C for 16 hours.
6) After the 16 hour exposure to 4°C, slowly peel each sample while it is in the cabinet to check for adhesion to the HDPE panel.
7) Test three specimens of each sample and report average result.

### Report

1) Report the substrate material.
2) Report the relative degree of adhesion to the substrate, excellent- being the best, very good- next best, good, fair, and poor being the worst (little or no adhesion).

### II-8 Melt Flow Index (MFI)

The melt flow index of the A-B-A block copolymers is measured according to ASTM-D 1238-95.
Report: Average of three samples in g/10 min.

### II-9 Specification of Standard Atmospheres for Conditioning and Testing

This measurement is according to ASTM-E 171-87

### III Test Results

The following table summarizes the results of the testing conducted on Examples 1 through 7 before thermal stability testing.

**Table 2**

| Test | Expl. 1 | Expl. 2 | Expl. 3 | Expl. 4 | Expl. 5 | Expl. 6 | Expl. 7 |
|---|---|---|---|---|---|---|---|
| Viscosity at 120°C (mPa•s) | 16,100 | 13,100 | 10,800 | 23,600 | 16,125 | 13,250 | 14,000 |
| Viscosity at 130°C (mPa•s) | 9,400 | 7,900 | 6,625 | 8,675 | 7,838 | 6,775 | 7,500 |
| Viscosity at 140°C (mPa•s) | 6,275 | 5,275 | 4,450 | 4,400 | 4,800 | 4,100 | 4,600 |
| Viscosity at 150°C (mPa•s) | 4,300 | 3,650 | 3,100 | 2,700 | 3,200 | 2,650 | 3,100 |
| RBSP (°C) | 89.4 | 87.3 | 85.3 | 99.8 | 93.7 | 88.1 | 92.2 |
| Initial Peel to Cotton (N/cm) | 1.7 | 1.9 | 1.6 | 1.95 | 1.65 | 0.8 | 0.7 |
| Staining/ Transfer to Cotton @ 40°C After 24 Hrs | None | None | None | None | None | Slight | None |
| Peel to Cotton After Aging at 40°C (N/cm) | 2.1 | 2.1 | 2.2 | 1.9 | 1.9 | 2.2 T | 1.1 |
| Aging @60°C after two weeks | Excellent | Excellent | Excell. | Excell. | Excell. | Poor | Very Good |
| Adhesion to HDPE @ 4°C | Excellent | Excellent | Excell. | Excell. | Excell. | Poor | Good |
| Coat Weight on PE Film (g/m2) | 19.0 | 18.2 | 20.3 | 18.1 | 20.2 | 19.7 | 20.1 |
| Expl.- = Example T = some transfer | | | | | | | |

**Table 3**

| The following table summarizes the results of the testing conducted on Examples 1 through 7 after exposure to 150°C for 48 hours, followed by 16 hours at 160°C. | | | | | | | |
|---|---|---|---|---|---|---|---|
| Test | Expl. 1 | Expl. 2 | Expl. 3 | Expl. 4 | Expl. 5 | Expl. 6 | Expl. 7 |
| Viscosity at 120°C (mPa•s) | 12,500 | 11,300 | 9,200 | 21,200 | 14,300 | 10,325 | 12,800 |
| Viscosity at 130°C (mPa•s) | 7,450 | 6,700 | 5,700 | 7,525 | 6,813 | 5,672 | 6,750 |
| Viscosity at 140°C (mPa•s) | 5,025 | 4,475 | 3,850 | 3,875 | 4,150 | 3,513 | 4,250 |
| Viscosity at 150°C (mPa•s) | 3,475 | 3,120 | 2,685 | 2,375 | 2,800 | 2,245 | 2,910 |
| % Change in Viscosity at 150°C | -19.1 | -14.5 | -13.4 | -12.0 | -12.5 | -15.3 | -6.1 |
| RBSP (°C) | 88.5 | 86.5 | 84.3 | 99.2 | 92.4 | 82.5 | 92.5 |
| Change in Physical Appearance | Minimal | Minimal | Minimal | Minimal | Minimal | Odor & Darker Color | None |
| Change in RBSP in (°C) | -0.9 | -0.8 | -1.0 | -0.6 | -1.3 | -5.6 | +0.3 |
| Residue to Cotton @ 40°C After 24 Hrs | None | None | None | None | None | Moderate | None |
| Coat Weight on PE Film (g/m²) | 21.1 | 18.2 | 19.4 | 19.9 | 20.3 | 21.1 | 18.9 |

## Claims

1. A hot melt pressure sensitive adhesive (HMPSA) comprising:
(a) about 10 percent to about 40 percent, based on the weight of the HMPSA, of a linear A-B-A block copolymer, wherein the B component is polyisoprene and the A component is a vinylaromatic polymer;
(b) about 40 percent to about 70 percent by weight of at least one compatible tackifying resin, based on the weight of the HMPSA;
(c) about 10 percent to about 40 percent by weight of at least one plasticizer, based on the weight of the HMPSA;
(d) about 0 percent to about 10 percent by weight of a wax, based upon the weight of the HMPSA; and
(e) about 0.1 percent to about 2.5 percent by weight of antioxidant(s) and stabilizer(s) based upon the weight of the HMPSA; and
wherein the A-B-A block copolymer contains 0 to 10 % by weight residual A-B diblock.

2. The hot melt pressure sensitive adhesive (HMPSA) of claim 1, wherein the adhesive has a viscosity from 3,500 mPas to 25,000 mPas at 140°C.

3. The hot melt pressure sensitive adhesive (HMPSA) according to claim 1, wherein the adhesive has an adhesion value from 1.0 to 3.0 N / cm at a coat weight of 10 to 25 grams per square meter.

4. The hot melt pressure sensitive adhesive (HMPSA) of claim 1, wherein the vinylaromatic polymer (A component) comprises about 15 percent to about 50 percent by weight of the A-B-A block copolymer.

5. The hot melt pressure sensitive adhesive (HMPSA) of claim 1, wherein the A-B-A block copolymer has a melt flow index of 3 to 50 g/10 min, determined by ASTM-d 1238-95.

6. The hot melt pressure sensitive adhesive (HMPSA) of claim 1, wherein the compatible tackifying resin is selected from the group consisting of aliphatic petroleum resins and the hydrogenated derivatives thereof, aromatic petroleum resins and the hydrogenated derivatives thereof, aliphatic/aromatic petroleum resins and the hydrogenated derivatives thereof, hydrocarbon resins, styrene resins, alpha-methyl styrene resins, polyterpene resins, copolymers and terpolymers of natural terpene resins, pentaerythritol esters of wood, gum, and tall-oil rosins, glycerol esters of wood, gum, and tall-oil rosins, mixed esters of rosins and mixtures thereof.

7. The hot melt pressure sensitive adhesive (HMPSA) of claim 1, wherein the plasticizer is selected from the group consisting of napthenic oil, paraffinic oil, phthalate and adipate esters, oligomers of polypropylene, polybutenes, polyisoprene, hydrogenated polyisoprene and polybutadiene, benzoate esters, vegetable, animal oils and derivatives thereof, and mixtures thereof.

8. The hot melt pressure sensitive adhesive (HMPSA) of claim 1, wherein the wax is selected from the group consisting of paraffin wax, microcrystalline wax, Fischer-Tropsch wax, polyethylene wax, ethylene vinyl acetate wax, oxidixed polyethylene wax, hydrogenated castor oil wax and derivatives thereof, polypropylene wax and mixtures thereof.

9. The hot melt pressure sensitive adhesive (HMPSA) of claim 1, comprising a mixture of a first linear A-B-A block copolymer having a polystyrene content of from 15 weight % to 22 weight % and a second linear A-B-A block copolymer having a polystyrene content of from 26 weight % to 32 weight %, the first and second A-B-A block copolymers each containing 0 to 5 weight % residual A-B diblock and a weight ratio of first linear A-B-A bock copolymer: second linear A-B-A block copolymer of from 1:2 to 2:1.

10. A disposable article, comprising:
i) a polyolefin or nonwoven substrate (i) and
ii) a pressure sensitive adhesive (ii) applied onto substrate (i), said pressure-sensitive adhesive comprising:
(a) about 10 percent to about 40 percent based on the weight of the HMPSA, of a linear A-B-A block copolymer, wherein the B component is polyisoprene and the A component is polystyrene;
(b) about 40 percent to about 65 percent by weight of at least one compatible tackifying resin, based on the weight of the HMPSA;
(c) about 10 percent to about 40 percent by weight of at least one plasticizer, based on the weight of the HMPSA;
(d) about 0 percent to about 10 percent by weight of a wax, based upon the weight of the HMPSA; and
(e) about 0.25 percent to about 2.0 percent by weight of antioxidant(s) and stabilizer(s);
wherein the A-B-A block copolymer contains 0 to 10 % by weight residual A-B diblock;
iii) wherein said pressure sensitive adhesive (ii) is bonding substrate (i) to at least one elastic, polyolefin, foam, or nonwoven substrate.

11. The disposable article of claim 10, wherein said pressure sensitive adhesive (ii) comprises:
(a) about 10 percent to about 40 percent based on the weight of the HMPSA, of a linear A-B-A block copolymer, wherein the B component is polyisoprene and the A component is polystyrene; and
wherein the A component is present in an amount of about 15 percent to about 35 percent by weight of the A-B-A block copolymer; and
wherein the A-B-A block copolymer has a melt flow index of 6 to 20; and
wherein the A-B-A block copolymer contains 0 to 5 % by weight residual A-B diblock.
(b1) about 40 percent to 70 percent of a hydrogenated aliphatic/aromatic tackifying resin;
(b2) about 0 percent to 10 percent of an endblock reinforcing resin;
(c) about 10 percent to 40 percent of paraffinic oil plasticizer;
(d) about 0 to 5 parts of an oxidized polyethylene wax or polyethylene wax or mixture of oxidized polyethylene wax and polyethylene wax; and;
(e) about 0.25 percent to about 2.0 percent by weight of antioxidant(s) and stabilizer(s);

12. The disposable article of claim 10, wherein said pressure sensitive adhesive (ii) has a viscosity of 3,600 mPas to 10,000 mPas at 140°C.

13. The disposable article of claim 10, wherein the pressure sensitive adhesive (ii) has an adhesion value from 1.0 to 3.0 N/cm at a coat weight of 10 to 20 grams per square meter.

14. The disposable article of claim 10, wherein the article is selected from the group consisting of plasters, bandages, sanitary napkins, adult incontinent pads or diapers.

15. The disposable article of claim 14, wherein the disposable article is a sanitary napkin for adhering to the crotch portion of an undergarment, comprising:
an adsorbent body for absorbing body fluids and having a body facing side and a garment facing side, wherein a layer of the pressure sensitive adhesive (ii) at a coat weight of 10 to 20 grams per square meter overlies at least a portion of the garment facing side, whereby said sanitary napkin may be applied to said undergarment and wherein the pressure sensitive adhesive exhibits an adhesion value of 1.5 to 3.0 N/cm and a transfer of adhesive of less than 1 milligram per square meter after being applied to cotton and conditioned for 24 hours at 40°C with 1 kg of pressure.

16. A method for the production of the disposable article of claim 10, wherein said pressure sensitive hot melt is applied by a method selected from the group consisting of transfer coating from silicone release paper or film, slot-die extrusion, fiber melt deposition, melt blown techniques and pattern coating methods.

17. The method of claim 16, wherein said pressure sensitive hot melt is applied directly to a polyolefin substrate using a technique selected from the group consisting of slot-die extrusion, fiber melt deposition, and spiral spray.
